# EUROPEAN PATENT APPLICATION

(11) **EP 0 925 794 A2**
(43) Date of publication of application: **30.06.1999**
(21) Application number: 98203641.0
(22) Date of filing: 28.10.1998
(51) Int. Cl.: A61L 2/10, A46B 17/06

(54) **Sterilizing device for toothbrushes**

(30) Priority: 16.12.1997 IT MI972778
(71) Applicant: Beghelli S.p.A., 40050 Monteveglio, Bologna (IT)
(72) Inventor: Beghelli, Gian Pietro, 40050 Monteveglio-Bologna (IT)
(74) Representative: Zanardo, Giovanni

(57) **Abstract**

A sterilizing device for toothbrushes (16) comprising a germicidal lamp (21) capable of destroying germs and microorganisms depositing on the bristles (35) of toothbrushes (16) during the teeth cleaning operation, which provides for a switch (17) to activate or deactivate the lamp (21), depending on the respective opening or closing of a container (10). The act of opening the container (10) consists in lifting up an upper section or cover (11) of the same, which consequently results, through a transmission mechanism (25) in a gradual tilting of a lower section (12) containing four seats (18) for as many toothbrushes (16), which are thus available to the user.

## Description

This invention refers to a container for toothbrushes equipped with a germicidal lamp, capable of destroying germs and microorganisms depositing on the bristles of the toothbrush.

The most effective means for destroying germs and microorganisms was until recent years represented by the ultraviolet energy radiated by the sun.

However, the ultraviolet or UV radiations reaching the earth's surface have a wavelength in the range of 1,900 to 3,600 Angstrom, with a modest bactericidal capability.

It therefore turned out to be necessary to arrange for a continuous flow of a large volume of air and extensive periods of exposure.

Experimental tests have demonstrated that the germicidal capability of ultraviolet radiations attains a maximum around the wavelength of 2,600 Angstrom.

The germicidal lamps emit about 25% of the energy absorbed at the wavelength of 2,537 Angstrom, with a bactericidal power considerably greater than that of solar radiations; they are for this reason optimally suited for a quick and effective disinfection.

The germicidal lamps have electrical and dimensional characteristics equal to those of fluorescent lamps of the same power rating.

They are constituted of a special quartz-based glass tube, capable of passing the ultraviolet radiations produced by an electrical discharge in mercury vapors, and carries two electrodes at its extremities; a reactor is charged with the function of regulating the lamp's current flow to the proper value, and a starter serves to preheat the cathodes and favor the priming.

The properties of germicidal lamps are such that despite their having become available in the current form only over the past twenty years, their applications have been greatly expanded.

In particular for the disinfection of toiletry articles such as toothbrushes, some radiating devices are currently used which are capable of guaranteeing an accurate control of the radations emitted by a germicidal lamp, so as to achieve a top performance.

On the other hand, an excessive exposure of human tissues to ultraviolet radiations, produces harmful effects, such as erythemas and conjunctivitis; it is therefore of fundamental importance that an apparatus or system based on germicidal lamps be free of any physiological risks for the persons likely to be exposed to the radiations.

For this purpose, numerous types of containers for toothbrushes, incorporating a germicidal lamp, have been designed.

However, they still exhibit certain drawbacks, such as a design configuration of the sterilizing device which is still entirely inadequate to ensure the user's proper safety against the risk of a potential exposure to the ultraviolet radiation, and presents the basic difficulty of accessing the inside of the container to remove the toothbrushes and a less than uniform exposure of the bristles of the toothbrush to the impinging ultraviolet radiation.

In particular, some toothbrush sterilizing devices are currently known, which are basically constituted by a housing providing seats for inserting the heads of each toothbrush, so as to turn the bristles toward the inside of the device, opposite an area covered by the impinging radiation of a germicidal lamp based on ultraviolet rays.

According to the preferred embodiments, after inserting the toothbrushes the user must slide a wall so as to confine the toothbrushes inside a compartment, thus impeding their removal during the operation of the device, operated by the user through a manual switch.

After shutting off the sterilizing device, also done by hand through a switching device, the sliding wall can be removed to extract the toothbrushes for their use.

The scope of this invention is therefore to indicate a toothbrush sterilizing device capable of eliminating the mentioned drawbacks, or to produce a container for toohbrushes with a built-in germicidal lamp presenting a geometric configuration and an arrangement of its mechanical parts capable of guaranteeing the user an effective safety against the skin's potential exposure to an impinging ultraviolet radiation.

Another purpose of this invention is to indicate a toothbrush sterilizing device capable of allowing a relatively easy access to the inside of the toothbrush container, so as to remove the toothbrushes while exempting the user from performing numerous and complex operations.

A further purpose of this invention is to produce a toothbrush container with a built-in germicidal lamp, whose ultraviolet radiation is efficient from an energy viewpoint, while totally and uniformly investing the entire area facing the toothbrush, where the bristles are exposed.

Not the least purpose of the invention is to produce a toothbrush sterilizing device capable of being produced easily and economically, without costly and complex technologies, by using standard circuit solutions and known electrical and electronic components.

These and other purposes are achieved by a toothbrush sterilizing device according to claim 1, which is being referred to for brevity.

According to this invention, a container for the disinfection of toothbrushes is advantageously mounted on the wall and may be opened by the user simply by lifting an upper portion or cover, so as to consequently allow, by a transmission mechanism, the gradual tilting of a lower portion and the user's access to the receptacle holding the toothbrushes.

At the same instant the user lifts the cover to open the container, a switch connected to the transmission mechanism deactivates the germicidal lamp, thus interrupting the power supply.

Alternatively, the sterilizing device returns to service when closing the container, thus guaranteeing the user's absolute safety.

The built-in electronic piloting circuit of the germicidal lamp, connected to a 230 V network and fed by transformer and signal modulating circuit is arranged in a vertical position, just above the bottom of the container fixed to the wall, and is protected by by an enclosure inaccessible to the user, so as to prevent any danger deriving from a contact with any parts of the electrical power supply circuit which may happen to be under power.

This structural arrangement of the radiating device's power feeding circuit substantially increases its electrical safety, especially in view of any malfunctioning of the system or small oversights on the part of the users.

For example, if the lower portion of the container designed to hold the toothbrushes and therefore necessarily fitted with one or more cavities is inadvertently or playfully filled with liquid, the user would not incur any risk, because he would in no case be in contact with the device's electrical components.

If the liquid should unfortunately seep in and come in contact with the lamp's power supply, the differential switch would spring into action and in the worst of cases the electrical power supply of the system would be cut off, thus again ensuring the user's complete safety.

Further purposes and advantages of this invention will become clearer from the following description and attached drawings, supplied for purely exemplifying and non limiting purposes, in which:

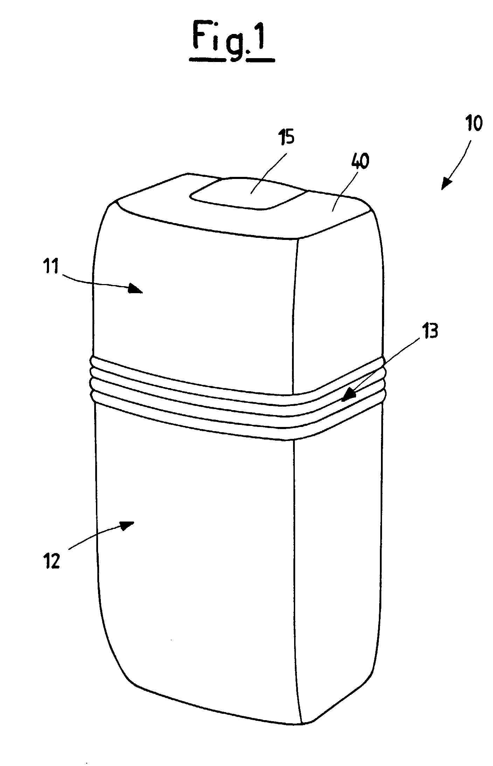

- Figure 1 is a prospective view of a toothbrush sterilizing device in a closed position, according to the invention;

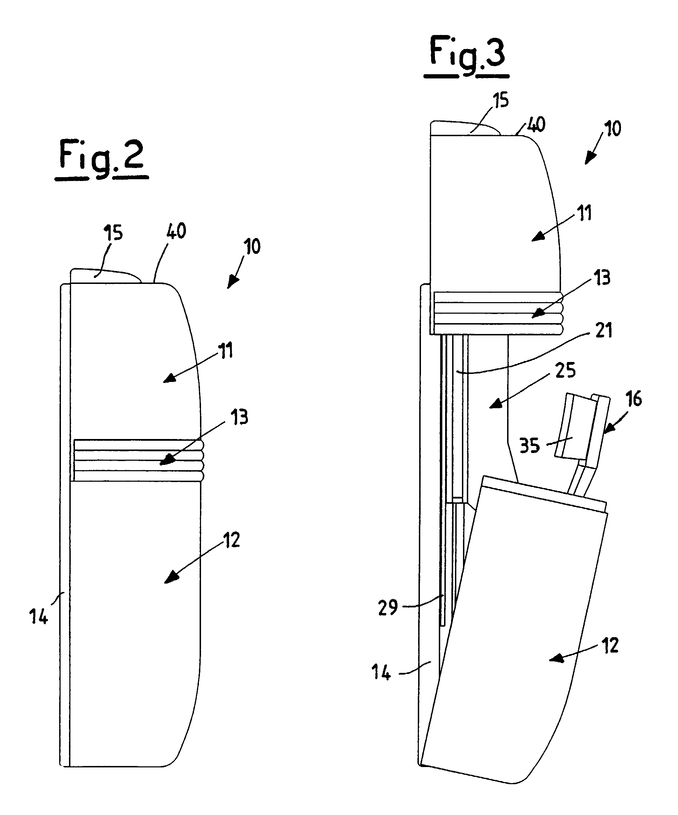

- Figure 2 is a side view of the device shown in Figure 1, in a closed position;
- Figure 3 is a side view of the device shown in Figure 1, in a open position;

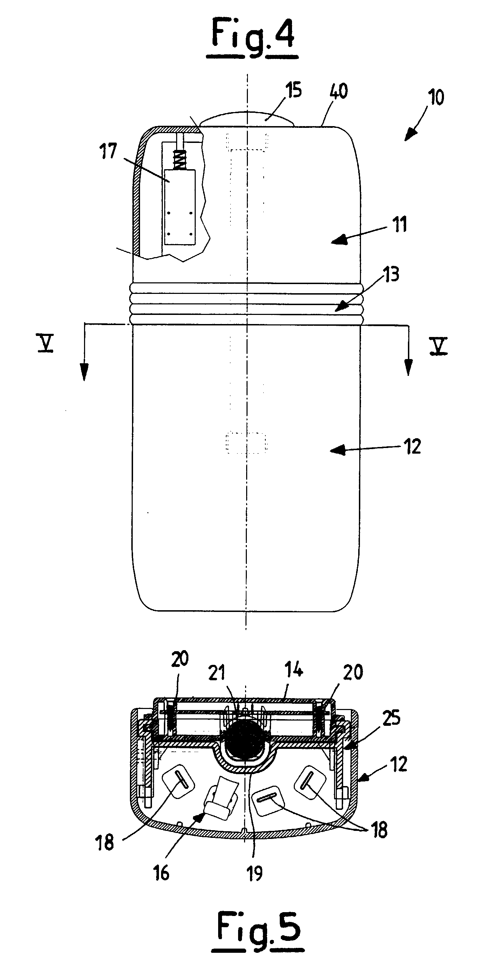

- Figure 4 is a frontal view of the device shown in Figure 1, in a partially sectionalized view, in a closed position, showing a deactivating switch for the germicidal lamp according to this invention;
- Figure 5 is cross-sectional view along a line V-V of Figure 4;

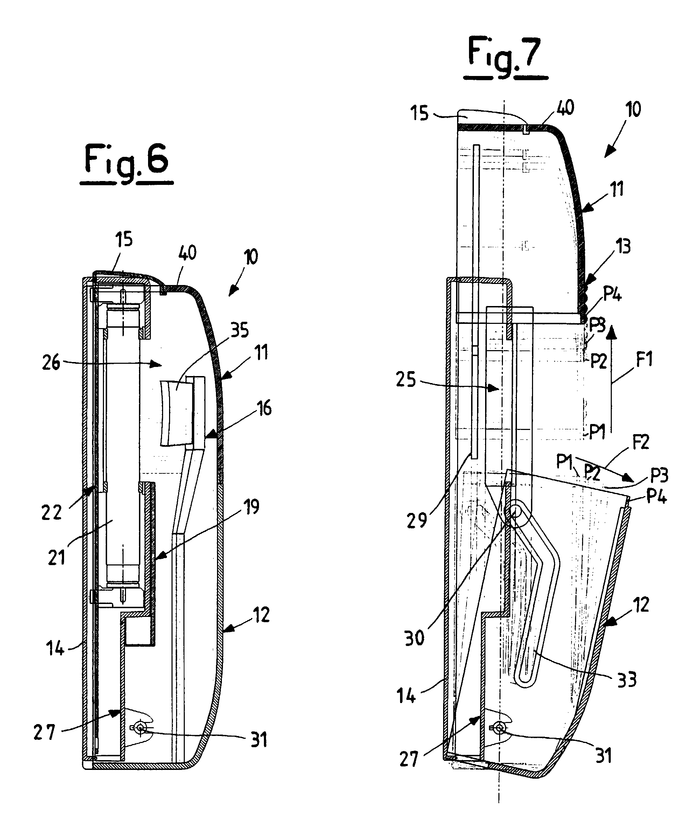

   Figure 6 is a cross-section of the toothbrush sterilizing device according to this invention, with the container in a closed position,
- Figure 7 is a cross-section of the toothbrush sterilizing device according to this invention, showing four different successive opening positions of the container.

With reference to the mentioned figures, a toothbrush sterilizing device according to this invention comprises a container, generally indicated by 10, essentially constituted by an upper portion or cover 11, delimited by a rim 13 separating it from a hollow lower portion 12, whose interior holds the toothbrushes 16, each set into a seat 18 of its own.

At the most, four seats 18 are provided for inserting a maximum number of four toothbrushes 16 in each sterilizing device.

The number 15 indicates a projection provided on the upper surface 40 of the container 10, while 14 indicates a bottom portion of a housing 27, containing an electronic piloting circuit 22 for a germicidal lamp 21, of a traditional type in itself known, which emits an ultraviolet radiation essentially covering the area, generally indicated by 26, where the bristles 35 of the toothbrush 16 fit themselves when the container 10 is closed.

The lamp 21 is protected by a wall 19 from any contact with the toothbrush 16.

In an exemplifying but non-limiting embodiment of this invention, the bottom portion 14 is wall-mounted, while the housing 27 of the electronic circuit 22 is blocked on the bottom 14 by four screws 20.

The number 17 indicates a switching device, preferably based on electronic components, which deactivates the sterilizing device, cutting off the electrical power supply from the 230 Volt power supply network to the electronic circuit 22, and therefore to the germicidal lamp 21, at the instant the cover 11 moves upward.

For the sake of simplifying the graphic representation, the electrical connection between the 230 Volt power supply network and the electronic circuit 22 is not shown in the attached drawings.

Alternatively, the electronic circuit 22 may be fed by an accumulator battery suplying a continuous current.

Finally, the number 25 generally indicates a transmission mechanism capable of moving the cover 11 and the lower portion 12 of the container 10, which essentially comprises a vertical sliding guide 29 of the cover 11 with respect to the housing 27 mounted on the wall with the aid of the bottom 14, a rotation pin 31 of the portion 12 of the container 10 and a small sliding roller 30 firmly affixed to the cover 11 by the transmission mechanism 25, which acts in a guide 33 provided inside the portion 12 while rotating it toward the outside, in the direction and rotating sense of the arrow F2, at the same time the cover 11 is lifted, in the direction and rotating sense of the arrow F1.

The operation of the toothbrush sterilizing device 16 is essentially as follows.

The bottom portion 14 of the housing 27 of the electronic piloting circuit 22 is preferably mounted on the wall and must be fitted with a hole to pass a power supply cable coming from the 230 Volts power supply network, using a plug connected to the circuit 22.

If the container 10 is closed and the plug is connected, the circuit 22 controls the germicidal lamp 21 to emit an ultraviolet radiation, at some pre-established frequency and intensity levels, so as to extend to a well defined radiating zone 26 to invest the brushes 35 of the toothbrushes 16.

The frequency and intensity values of the impinging radiation depend on the surface to be irradiated, the distance between the light source and the object to be irradiated, and the dimensions of the object itself.

It should however be noted that in this case, once the structural design of a particular type of sterilizing device has been adopted, these values are predetermined and constant, being memorized inside the microprocessor of the circuit 22 at the time of manufacturing the product.

If the user needs a toothbrush 16 and wants to remove it from its seat 18, or if he simply wishes to cut off the sterilizing action of the germicidal lamp 21, it suffices to lift the cover 11, in the direction pointed by the arrow F1.

As soon as the cover 11 and the rim 13 are detached from the lower portion 12, the switch 17, being triggered by a proximity transducer, deactivates the lamp 21, cutting off the electrical power supply to the circuit 22 so that no ultraviolet radiation can be emitted toward the outside in any manner which upon contact with the human skin may emit noxious or even harmful effects.

Figure 7 represents the opening mechanism of the container 10 in detail; during the upward sliding action (direction of the arrow F1) of the cover 11, along the guide 29, which is firmly affixed to the same cover 11, the lower portion 12 moves in the direction of the arrow F2, around the fulcrum 31.

Figure 7 illustrates four intermediate positions, indicated by P1, P2, P3 and P4, in which the container 10 passes from a minimum (P1) to a maximum (P4) opening position.

The upward motion of the cover 11 is transmitted, through the transmission mechanism 25, to a sliding roller 30, which is firmly affixed to the mechanism itself (26) acting inside a guide 33 provided in a cavity of the lower portion 12.

As the cover 11 moves upward in a vertical direction, the mechanism 25 slides the roller 30 above the knee of the guide 33; the upward motion of the roller 30, which slides along the contoured walls of the guide 33, forces the entire portion 12 to rotate toward the outside in the direction of the arrow F2, around a center of rotation constituted by the hinge or fulcrum 31.

The total opening of the container 10, which assumes a position indicated by P4 in Figure 7, allows an easier access, with respect to the known devices, to the internal cavity of the portion 12, thus making it possible for the user, depending on his needs or requirements, to insert the toothbrushes 16 inside their special seats 18 or remove them from the same, without any risk of being invested by the UV radiation emitted by the lamp 21.

In the same manner, a malfunctioning of the system or any accident due to chance or inexperience cannot turn into a more serious event; for example, the introduction of water or other liquid inside the cavity of the portion 12, made by chance or play, may, in the worst case, cause an interruption of the power supply to the system the sterilizing device is connected to, while in any case protecting the user from any electrical risk.

When the cover 11 is again pushed down, the roller 30 will consequently slide down below the knee of the guide 33, forcing the portion 12 of the container 10 to rotate around the fulcrum 31, in the direction of the arrow F2 and in the opposite sense, until it comes to a position on which the bottom wall of the portion 12 is parallel and adjacent to the bottom 14.

In this position the rim 13 of the cover 11 contacts the upper surface of the portion 12, and the switch 17 instantly reactivates the lamp 21, thus allowing the electrical feeding current to flow from the network to the electronic circuit 22, over a transformer and signal modulating circuit.

The above description clarifies the characteristics as well as the advantages of the toothbrush sterilizing device as an object of this invention.

In particular, these are represented by:
- ease of access to the space holding the toothbrushes;
- instant deactivation of the emission of ultraviolet radiation when opening the container and safety for the user against electrical risks due to system failures, casual accidents, and electric network power outages;
- observance of the existing national and international regulations in matters of electrical safety;
- limited cost with respect to known devices.

Finally, it is obvious that numerous other variations may be applied to the toothbrush sterilizing device object of this invention, without thereby abandoning the principles of novelty inherent in the inventive concept, and it is equally clear that in the practical implementation of the invention, the materials, shapes and dimensions of the illustrated details may be of any kind, depending on the requirements, and that the same may be substituted by others of a technically equivalent nature.

## Claims

1. Sterilizing device for toothbrushes (16), of a type comprising a container (10), constituted by a multiple number of portions (11, 12), where at least one (12) of said portions (11, 12) holds the toothbrushes (16) each inserted in a seat (18), whose bristles (35) are invested by an ultraviolet radiation emitted by at least one source (21) controlled by an electronic piloting circuit (22) supplied by an electrical power feed, characterized in that said portion (11, 12) constituting said container (10) is connected to at least one transmission mechanism (25) comprising a fixed portion provided on a housing (27) of said electronic circuit (22) and a mobile portion firmly attached to one (11) of the said portions (11, 12) of the container (10), in such a manner that a vertical motion (F1) of the first portion (11) of said container (10) corresponds to an inclination (F2), toward an external user, of the second portion (12) of said container (10), for the purpose of opening said container (10) and accessing the toothbrushes (16) contained therein, whereby the positions (P1, P2, P3, P4) assumed by said portions (11, 12) of the container (10) are reached continuously within a predetermined set of values.

2. Sterilizing device according to claim 1, characterized in that, at the same instant of opening said container (10), at least one switching device (17) deactivates said sterilizing device while cutting off the electrical power supply to said electronic piloting circuit (22) of said source (21) emitting ultraviolet radiations, and that said switch (17) reactivates said sterilizing device at the instant said container (10) is closed.

3. Sterilizing device according to claim 1, characterized in that said fixed part of the transmission mechanism (25) comprises at least one sliding roller (30) firmly affixed to said second portion (12) of the container (10), while acting on at least one guide (33) provided inside said second portion (12) of the container (10) and rotating it toward the outside by the action of at least one fulcrum (31) connected to said housing (27) of said electronic circuit (22).

4. Sterilizing device according to claim 1, characterized in that said mobile part of the transmission mechanism (25) comprises at least one vertical sliding guide (29) firmly attached to said first portion (11) of the container (10).

5. Sterilizing device according to claim 1, characterized in that said housing (27) of the electronic circuit (22) is attached by fastening means to at least one bottom (14) of said housing (27), which is in turn attached to the wall.

6. Sterilizing device according to claim 1, characterized in that said source (21) emitting ultraviolet radiations is constituted by a germicidal lamp, emitting ultraviolet radiations in an area (26) facing said bristles (35) of the toothbrushes (16).

7. Sterilizing device according to claim 1, characterized in that it comprises at least four seats (18) arranged in the second portion (12) of the container (10) of an internally hollow shape suitable for inserting an equal number of toothbrushes (16).

8. Sterilizing device according to claim 1, characterized in that said enclosure (27) of the electronic piloting circuit (22) extends vertically along the larger dimension of said container (10) and behind said source (21) of ultraviolet radiation.

9. Sterilizing device according to claim 1, characterized in that it provides, in front of said source (21) of ultraviolet radiation, a protective wall (19), extending for at least a portion of the length of said source (21).
